(19) Europäisches Patentamt
European Patent Office
Office européen des brevets

(11) **EP 2 577 242 B1**

(12) **EUROPEAN PATENT SPECIFICATION**

(45) Date of publication and mention
of the grant of the patent:
**24.10.2018 Bulletin 2018/43**

(51) Int Cl.:
***G01J 5/00*** *(2006.01)*  ***G01J 5/02*** *(2006.01)*
***G01J 5/06*** *(2006.01)*

(21) Application number: **11735544.6**

(22) Date of filing: **03.06.2011**

(86) International application number:
**PCT/IB2011/052443**

(87) International publication number:
**WO 2011/151806 (08.12.2011 Gazette 2011/49)**

(54) **METHOD AND DEVICE FOR MEASURING THE INTERNAL BODY TEMPERATURE OF A PATIENT**

VERFAHREN UND VORRICHTUNG ZUR MESSUNG DER KÖRPERINNENTEMPERATUR EINES PATIENTEN

PROCÉDÉ ET DISPOSITIF DE MESURE DE LA TEMPÉRATURE CORPORELLE INTERNE D'UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2010 IT MI20101531**
**04.06.2010 IT MI20101007**

(43) Date of publication of application:
**10.04.2013 Bulletin 2013/15**

(73) Proprietor: **TECNIMED S.r.l.**
**21040 Vedano Olona (Varese) (IT)**

(72) Inventor: **BELLIFEMINE, Francesco**
**I-21100 Varese (IT)**

(74) Representative: **Ponzellini, Gianmarco et al**
**PGA S.P.A., Milano**
**Succursale di Lugano**
**Viale Castagnola, 21c**
**6900 Lugano (CH)**

(56) References cited:
**DE-B3-102004 027 443    US-A1- 2004 254 472**

## Description

The present invention relates to a method and an apparatus for measuring a body temperature of at least a subject.

In particular the present method and the present apparatus are particularly applicable in measuring a body temperature of a plurality of subjects who are in or are crossing a determined area, such as a passage area in an airport or a room in a hospital, or another public area.

In a further aspect, the present method enables reliably detecting a temperature of numerous subjects in a cold environment or coming from an environment in which the temperature is not known.

As is known, in recent years the need to be able to measure a body temperature of a plurality of subjects, rapidly and non-invasively, has assumed a very considerable importance.

The growing and widespread fear of pandemics (for example, SARS, avian influenza or swine 'flu) has made it necessary to carry out more effective controls on transiting subjects, in particular those entering a determined company, area or place.

Think for example of airports, intensely crowded areas of passage for people in arrival from a very wide variety of countries, who can easily bring a rapid diffusion of contagion.

In this situation, in past years controls have been more widely made in airports, especially with reference to subjects in arrival from countries at risk.

It is however clear that measuring the body temperature of a subject at a time in an area of transit and passage for such a large number of people is quite impracticable and hampers smooth and fluid through-put in the airport.

For this reason, even the use of non-invasive and sufficiently rapid temperature detecting thermometers have been shown not to be ideal in preventing delays, queues and congestion in control areas, although the success of their use is evident in detecting subjects having high temperatures.

In the light of the above-evidenced problems, studies have been made and solutions have been proposed involving thermal imaging cameras using the infrared spectrum which can detect the radiation coming from a determined area under control and, thanks to the intensity of this radiation, which can also set up a map of temperatures in the zones placed under control.

This detection activity can also be done continuously or dynamically in such a way that the passage of a flow of subjects can be examined in real time.

In other terms one or more thermal imaging cameras control access zones transiting subjects have to pass through; these cameras use the infrared spectrum of radiation received to assess the temperature gradients in the area under control.

The above-mentioned thermal imaging cameras enable continuous and extremely rapid control on a plurality of transiting subjects, but are affected by some drawbacks and/or operating limitations.

Firstly, known thermal imaging cameras necessarily require the presence of operators in order to identify subjects with fever.

It is at present not possible to automate the decision process, i.e. distinguishing which subject has a high temperature from a subject running a normal temperature, without the help of one or more operators. Typically the temperature profile is made using colours which go from dark (lower temperatures) to livid red or white (higher temperatures).

In some applications it is possible to select a determined subject and in particular a point of the detected image, and automatically obtain, using a conversion software, the temperature measured at that point, or the maximum temperature internally of the area of detection.

However the maximum temperature in the area of detection may not necessarily be the effective temperature of a subject as it may be due to the subject's carrying a cup of coffee, or using a mobile telephone, or holding a lit cigarette.

On the other hand, in systems where the maximum temperature internally of the considered area is evidenced, it can happen that several subjects are presenting high temperatures but the system would detect only the one with the highest temperature, while the others would be effectively ignored.

It should also be noted that with traditional contactless thermometers it is not at present possible to reliably detect the temperature of subjects coming from areas of temperature where the temperature is not known or not controllable.

In these situations it is not known (or not implemented in the architecture, i.e. the memory, of the device) which corrective parameter is to be applied to the body temperature detected in order to obtain the internal temperature.

Further, the temperature of a subject who is in or coming from an area having a low ambient temperature can also create problems in correctly detecting, as the more different the temperature of the target surface from the internal surface (obviously the cooler the external ambient the greater the difference) the less reliable the correction of the detected temperature is.

Document DE102004027443 B3 discloses a measurement system for contactless body core temperature determination using an evaluation unit for evaluating infrared signal that selects maximum value in a matrix and a computer unit for computing core temperature from selected maximum value. The measurement system has a matrix-shaped infrared sensor directed at the eye angle area on the nose side (canthus). The evaluation unit evaluates the infrared signal selecting the maximum value in the matrix and the computer unit computes the core temperature from the selected maximum value and outputs the computed value.

It is also known from document US 2004254472 an approach to noninvasively, remotely and accurately detect core body temperature in a warm-blooded subject, human or animal, via thermal imaging. This document describes to acquire thermal image data for one or more subjects and then using the thermal image data to derive temperature information about the subject(s). Thermal imaging data are acquired by using a digital thermal image capture device (thermal camera). The use of in-frame temperature references, specific anatomical target regions namely, the forehead and the region of the eyes, and a physiological heat transfer model help to overcome pitfalls inherent with other thermal imaging techniques applied to physiological screening applications. This document teaches to noninvasively, remotely and rapidly screen for diseases or conditions that are characterized by changes in core body temperature. One human application of this teaching is the remote screening for severe acute respiratory syndrome (SARS), since fever is a common, early symptom.

In this situation the technical aim underpinning the invention is that of obviating the above-evidenced drawbacks.

A first aim of the invention is to make available a methodology as described in claim 1 and an apparatus which enable a more accurate identification of the subjects affected by fever with respect to healthy subjects, eventually also enabling automation of this process.

A further aim of the invention is to enable more precise measurements to be made, by eliminating systematic errors due to the main environmental effects.

A further aim of the invention is to disclose an intelligent apparatus which has costs that are entirely comparable with thermal imaging cameras at present available on the market, and possibly even lower, while guaranteeing performances that are very considerably improved.

A further aim of the invention is to improve the reliability of the determination of the internal temperature, even in situations of potential uncertainty in relation to the ambient temperature in which the subject as stabilised her or his own body temperature or in a case where the subject(s) is/are in particularly cold environments, such as for example prior to entry into public places such as schools, hospitals, factories, etc.

These and other aims are substantially attained by a method and a relative apparatus for determining the body temperature of at least a subject, according to the accompanying claims.

Further characteristics and advantages will more fully emerge from the detailed description of a preferred though not exclusive embodiment of a method and an apparatus according to what is described herein below.

The description is made in the following with reference to the accompanying figures of the drawings, provided by way of non-limiting example and in which:

figure 1 is a schematic view of an embodiment of an apparatus according to the invention;
figure 2 is a view by way of example of an image detected using a thermal imaging camera according to the present invention;
figure 3 is a contactless infrared thermometer;
figure 4 is a schematic view of a wave guide usable in the thermometer of figure 3;
figure 5 is a block diagram relating to a measuring method which exploits the thermometer of figure 3; and

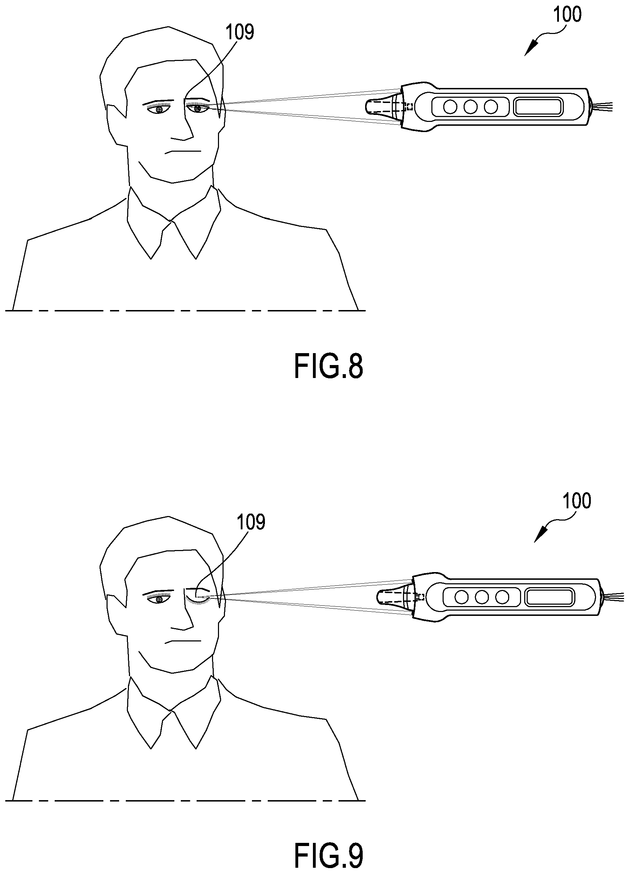

figure 6 is an alternative embodiment of the apparatus of figure 1; figures 7, 8, 9 illustrate the thermometer of figure 3 in a respective measuring condition of a patient.

With reference to the figures, 1 denotes in its entirety an apparatus for determining a body temperature of at least a subject S.

In particular, the apparatus is normally called a thermal imaging camera, or thermal imaging camera, and is a special television camera that is sensitive to infrared radiation and able to obtain thermographic images or films.

In other terms the device is able to acquire at least images in the infrared field and to two-dimensionally visualise the radiating measurements.

In general, with the use of a thermal imaging camera non-destructive and non-invasive controls are made as radiations in the infrared field of the electromagnetic spectrum are detected, and measurements are performed that are correlated with the emission of the radiations.

In particular the thermal imaging camera is able to detect temperatures of human bodies analysed by means of measuring an infrared intensity emitted by the bodies under examination.

All objects at a temperature of higher than absolute zero emit radiations in the infrared field and the relation is numerically quantifiable thanks to Planck's law.

In general, thermography enables visualisation of absolute values and temperature variations of people or objects, independently of the illumination thereof in the visible field.

The quantity of radiation emitted increases proportionally at the fourth power of the absolute temperature of an object, as clearly established by the Stefan-Boltzmann law which defines the correlation between radiation and temperature according to the following formula:

$$q = \varepsilon \sigma T4$$

where $\sigma$ is the Stefan-Boltzmann constant and $\varepsilon$ is the emissivity of the emitting surface (variable between theoretical limits of 0 and 1), while T is the absolute temperature.

Starting from the radiation detected, temperature maps of the exposed surfaces are thus obtained.

The image is in general constructed on a matrix of a certain number of pixels and a certain number of lines and the electronics of the instrument are able to rapidly read the energy value stored by each single pixel and to generate a corresponding image, in black and white or in false colours, of the observed object.

The spectral field of functioning of the thermal imaging camera is, as mentioned, the infrared field and in general the band most commonly used and most suitable for measuring values close to ambient temperatures of interest is the far infrared band (LW)

Overall, a thermographic system or IR is constituted by a television camera (thermal imaging camera) 1 connected to (or which includes on board) a processing and recording system of the images.

The infrared detectors have the task of identifying the consistency of the radiation which strikes them and of analysing, point by point, the radiating surface, in order to reach a definition of the thermal map.

From the structural point of view the apparatus 1 for calculating the body temperature of at least a subject S comprises an acquisition system 2 of an image relating to at least a predefined area 3 internally of which at least one and preferably a plurality of subjects S are located (or pass through), whose body temperature is of interest.

The apparatus obviously comprises the appropriate optical means 5 for enabling focusing and acquisition of the images; these means 5 are constituted by lenses or tele-objectives of a standard type and chosen according to the specific application to which the thermal imaging camera will be destined, as will be more fully explained in the following.

The apparatus further comprises an infrared sensor 6 which can be, merely by way of non-limiting example, a flat matrix microbolometer sensor, or a single matrix sensor, or a dot matrix sensor.

The sensors 6 can be non-cooled or cooled (in general with Peltier cells or a Stirling pump).

Alternatively, and still non-limitingly, sensors can be used having a thermopile matrix with configurations suitable for the particular situation: single-line (for example 8 elements) with mechanical scanning of the image, or matrix (for example 4x4, 16x16 or 32x32 lines per column). In this last case (matrix) the number of elements identifies the pixel resolution (16 lines per 16 columns = 256 pixel).

The material constituting the sensor influences the thermal sensitivity of the thermal imaging camera, i.e. the tiniest temperature difference detectable by a thermal imaging camera.

It is clear that a high level of thermal sensitivity identifies the most imperceptible differences of temperature, and therefore the smaller the value, the greater the measurement resolution of the thermal imaging camera and the quality of the image.

Merely by way of example, thermal imaging cameras having a thermal sensitivity of at least 80 mK can be used.

Obviously the field of measurement of the temperature of the apparatus for determining the body temperature of the invention must be centred in the range of the normal temperature of the human body, i.e. between 30 and 45 degrees Celsius with the best performances bracketed at around 37°C.

Since the apparatus of the invention will in general be destined to acquire images of subjects in motion or in any case must be able to perform also that type of scanning, the frequency of acquisition is a fundamental parameter for the use of the thermal imaging camera.

The measurement of the temperature of an object in motion must be performed with a good degree of frequency in order to avoid the phenomenon of smearing on the image, which prevents an accurate temperature measurement.

In general acquisition frequencies of greater than 60 Hertz are preferred. The instrument therefore also comprises a processing unit 7 able to acquire, with the appropriate frequencies, the electric signal emitted by the infrared sensor 6 following the acquisition of the image or the thermal map.

The same processing unit 7 is able to process the received signal and to show the thermal image detected on a suitable display 8 present in the apparatus (or in an external system - a computer for example).

In particular it is suitably possible to select the (false) colours for display that are linked to the different temperatures with the desired degree of precision.

In other terms it is possible to visualise, with different colours, variations of temperature from 30 to 43 degrees, for example, with colder colours for the lowest temperatures up to warmer colours for the higher temperatures.

With the device it is possible to measure the temperature of each single dot of the image; the instrument (or in any case the processing software during post-processing) includes a module 9 able to correlate radiation and temperature by means of the Stefan-Boltzmann law.

Note that the device further comprises a real image sensor 10 that can acquire the real image relating to the predefined area 3 which contains the subjects whose bodily temperature is to be detected.

In other terms, the thermal imaging camera of the invention further comprises a common sensor 10 of a type at present used in photographic cameras or video cameras present on the market that are able to acquire the image of the visible spectrum (visible light).

The detected signal is acquired by the processing unit 7 and can be shown on the display 8 both as an alternative to the thermographic image and together therewith, in a superposing fashion.

In particular, for each infrared image detected a real digital image is also automatically recorded.

In this way the system always provides two images that are corresponding and contemporaneous.

The apparatus can optionally comprise suitable integrated LEDs or a further lighting system 11 which guarantee optimal visibility in the image acquisition zones 3.

The processing unit 7 is advantageously capable of analysing the acquired image by means of a suitable recognition module 12, for identification of at least a zone of interest 13 of the subject S.

In detail, the instrument is able to acquire the image originating from the predefined area 3 and to perform on the acquired image a predetermined number of calculations which are capable of performing the recognition of the zone of interest 13 of the subject S.

In particular it is preliminarily advantageous to be able to identify, for each of the subjects of interest, one or more selected zones (for example) from among the forehead, eyes, temples, neck, ear auricles, the ethmoid sinus, the canthus, etc.

Note that the recognition operation could also be carried out alternatively on the infrared image acquired with detail by the sensor 6 (in this case the sensor of real images 10 in the spectrum of visible light could be totally optional) or also might perform the recognition directly on the real image acquired by the second sensor 10.

This second option is today commercially more advantageous as there exist methodologies which already use face recognition of a subject, included in photographic cameras and video cameras, even those used at amateur level.

In general the teachings, for example, of one or more of US patents US 6940545, US 7218759 or US 2009/0190803 can be used; these enable analysis of the image for identification in the zone of interest.

In any case a recognition operation carried out using the thermal imaging camera directly, i.e. without the use of the real image captured in the visible, but by using the thermal image, enables exploitation of the hardware already present in the device without requiring more.

The recognition methods can be different according to needs and also possibly according to the place of specific application of the thermal imaging camera.

Purely by way of example, firstly an oval - of the face (generally warmer) - can be distinguished with respect to the background (an environment which is usually at a temperature of lower than 37 degrees). Further, it is possible to recognise the positioning of the eyes internally of the oval, and possibly even of the lips and/or the nose.

In any case, once the thermographic method has identified some significant portions of the face, it is possible to establish, with reasonable precision and very rapidly (as well as automatically) the position of the area where the infrared radiation for reliable measurement of temperature can be sourced.

In a case of fever, cold or influenza, some portions of the face are hotter and change not only the temperature but also the relative distribution of the various temperatures on the face.

The clinical situation of the subject might therefore also be determined by not (or by not only) detecting the temperature of the portion of interest, but also the extension of a hotter area and/or a temperature delta between different portions of the face beyond or below a predetermined threshold (for example temperature differences between the nasal septum and the forehead that are greater than a standard value).

Obviously the interest for the present invention in this specific case is to identify not so much the face of a subject but rather the above-mentioned specific zones of interest, which are the best for the subsequent thermal analysis.

Once the specific zone of interest 13 is identified, the analysis of the infrared radiation is performed on it.

The determination of the subject's body temperature is calculated as a function of the infrared radiation originating from the zone of interest.

In general calculation is made, with specific reference to the zone of interest (for example the forehead), of the radiation of maximum intensity originating from the zone of interest and then the body temperature is calculated as a function of the maximum radiation detected.

The importance of the determination of the effective zone of interest of the subject is linked to the possibility of improving the accuracy of the measurements and also the possibility of automating the calculation of the temperature of a subject without error.

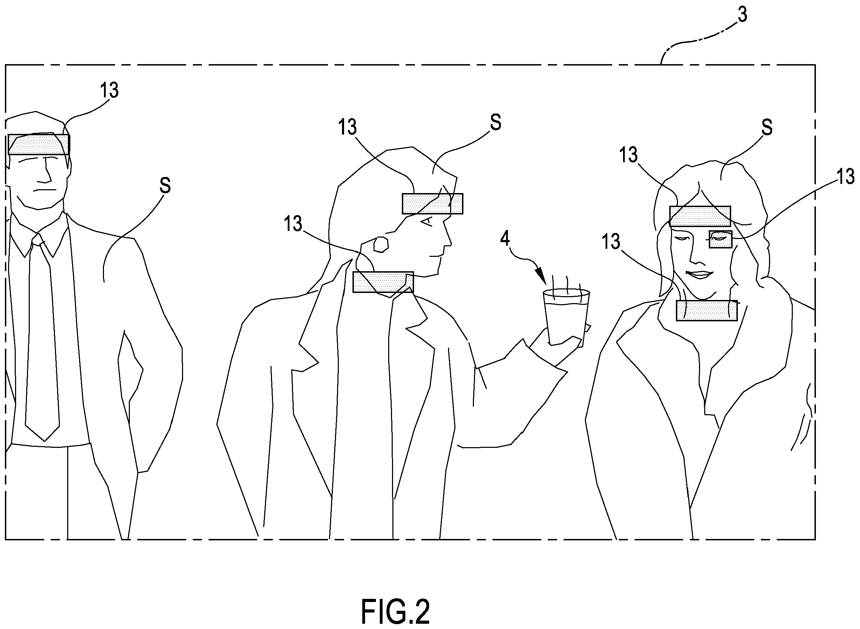

The recognition of the zone for which the temperature is to be detected enables avoiding that the software can be deviated into calculating and signalling temperatures outside the predetermined range, linked to sources of heat which are of no interest (see zone 4 in figure 2).

Consider, for example, hot drinks carried by subjects, or electronic apparatus that heat up, or even simply a cigarette, which would lead to the detection of high temperatures of no interest to the intended detection.

Thus, the identification of the precise zone 13 at which to perform the measurement, thanks to the recognition software, primarily enables elimination of the above-cited problematics.

Further, the methodology of the invention also comprises a step of correcting the temperature detected in order to determine the effective body temperature.

Firstly, the step of correction will be (or can be) a function of the zone of interest originating the infrared radiation.

It is in fact clear that the origin of the infrared radiation coming from the forehead, rather than from the neck (jugular artery) or the temples can require a different corrective parameter.

Note in fact that in general the external temperature of the various portions of the body not only differs from the internal temperature but also differs in a considerably different way according to the point of detection, because of a different blood supply or a different exposure to air which contributes to dissipating heat (the temperature of the forehead is generally lower than the temperature of the temples, and so on).

Note also that this correction can be performed, alternatively, with corrective functions or even with parameter tables having fixed correction data, obtained also through experimentation.

A second correction, advantageous or necessary, is instead a function of the ambient temperature in which the subjects whose temperature is to be measured are situated.

A subject having a same internal temperature which is in an environment having a low temperature will have a surface body temperature that is lower than the same subject having the same internal temperature who is situated in a warm environment.

Therefore, a first corrective parameter might be linked to the ambient temperature of the environment in which the subject is located at the moment of the detection or even at the ambient temperature (different from that of the detection area) in which the subject is located for a predetermined time interval such as to substantially influence and stabilise the temperature of interest immediately prior to detection.

Consider for example subjects whose temperature is detected at the entry to a climate-controlled area and who are coming from an area having a different temperature.

A subject who is outside in cold temperatures and then enters through an entry at the moment his or her temperature is measured will obviously have his or her temperature stabilised with reference to the outside temperature and not with reference to the place in which the measurement takes place.

In this case the ambient temperature used as a corrective reference can be the external temperature.

Further, a corrective step can also take account of an expected detection temperature predetermined previously for a majority of the plurality of subjects.

In other terms, without intervening with corrections linked to environmental parameters or detection zones 13, a thermal detection is performed on a plurality of subjects who are, on the whole, expected to be in good health (body temperature of about 37°).

In this case the temperature detected for this plurality of subjects will be corrected in such a way as to assume the value of 37° as the internal temperature of the majority of subjects presenting a same thermal radiation emitted from the corresponding zone of interest and then the differences with respect to subjects presenting higher temperatures will be evaluated.

In other terms, again it is supposed that the majority of the subjects whose temperature is detected will have a normal value and therefore, should the detected temperature for these subjects be, for example 35°, it would be corrected to 37°, automatically correcting all the other temperatures too. Naturally it is expected that the mean of the real temperatures of the subjects will change repeatedly and continually during the course of the day due to variations in the temperature in the external zone, and therefore the corrective value will be updated, for example every minute, by calculating the mean of the real temperatures of the subjects transiting over the last minute, or every five minutes, or even every "x" number of subjects.

In this sense it might also be possible simply to highlight those subjects who present a temperature, in the body zone of interest, that is higher than a determined threshold, with respect to the average of the subjects whole temperature has been detected during the designated time interval.

In other terms, instead of determining a precise body temperature in order to establish the presence of subjects with a fever, it is considered that the majority of subjects whose temperature is read do not present feverish phenomena and only those subjects whose relative temperature is higher are signalled.

The above is obviously done with reference to detections connected to the identified zone of interest, which is the same for all subjects, in such a way as to be reliable and automatable.

Note that the above-described methodology is particularly suited to temperature detections in areas where there is much passage of people, such as for example airports, railway stations, port areas, factories, schools, museums, hospitals (in the last case with particular reference to visitors).

One or more thermal imaging cameras positioned at compulsory accesses, for example passengers entering a country, enables measuring the internal temperature of a plurality of subjects reliably and signalling, in real time, those who might be affected by feverish pathologies.

The device can also be associated to an automatic access control system, for example constituted by a gate or a turnstile which automatically blocks to prevent passage of a subject "under suspicion". In this case the subject is sent to a special area where a specialised operator will carefully assess his or her temperature using more traditional systems and will possibly medically examine the subject.

Operating as described above, the apparatus is capable of eliminating the false positives connected to high temperatures in subjects of no interest, and even of performing precise measurements thanks to the corrective steps as described above.

Clearly, a further possible application of the thermal imaging camera is in hospitals or any public place such as to detect the temperature of the visitors and/or those passing through.

For example the thermal imaging camera can be positioned at accesses to hospital departments with patients in critical conditions, such as to be able to selectively prohibit entrance to visitors having fevers.

In a further advantageous aspect of the invention, though of secondary importance, a step of recognition of a face and/or a further bodily characteristic of a determined subject can be included, such that by a comparison with faces and/or other corresponding and pre-memorised bodily characteristics it is possible to identify the subject.

In this way it is not only possible to detect a determined temperature, but also to associate it to a specific subject/patient identified.

A possible application of this method would be extremely advantageous in hospitals or health care structures.

It is in fact possible to memorise faces or in any case identifying parameters of determined patients/subjects who can thereafter be recognised and/or identified by means of the thermal imaging camera. For example a worker or a student who normally presents a higher or lower temperature than a value Y with respect to the norm without being affected by any pathology could be recognised and allowed to pass automatically without being each time forced to undergo a medical examination, on the condition that the difference in temperature is normal for him or her.

A thermal imaging camera positioned in a room in a hospital might be able to detect not only the temperature of a subject but also, for example, his or her face, thus identifying the patient and automatically memorising his or her temperature. If the device is positioned at the bed it could continuously monitor the patient's temperature.

In these cases the devices might be automatically able to emit an alarm to signal patient/s with fevers at the moment in which the fever presents, i.e. at the moment when the body temperature exceeds a determined control threshold. This threshold can also be a low temperature, for example in a case of a deceased patient.

The system can also be associated to traditional control systems used by security personnel such as for example controls using metal detectors, X-ray sensors or equivalent systems in airports, tribunals etc.

In these cases a subject presenting a "suspect" temperature alerts the relative alarm signal and is sent to a special area where medical personnel can carry out the necessary medical checks.

In a simpler and alternative embodiment to the one described, an articulated structure 201 is realised, or even one constituted by a single fixed panel 202 (or substantially fixed) which each subject so requested must approach.

Observing the panel directly and frontally at a predetermined distance (about 50 cm) the panel 202, the infrared radiation coming from the zone of interest (for example, though not limitedly, the forehead) and the body temperature of the subject is received and calculated from this radiation.

In particular the correct distance can be signalled by the device in order to detect the temperature 200 using signalling means 203 (for example a beeping microphone or one or more LEDs 205); by way of example, luminous means and/or ultrasound means 206 could be used to establish whether the subject has reached the optimal position for the measurement to be taken.

When the subject is at the right distance, the infrared sensor 207 mounted on the panel 202 detects the radiation coming from the subject and then a control unit 208 calculates the body temperature as a function of the radiation detected and the ambient temperature. In other terms, the real internal temperature will be obtained by correcting the temperature detected from the forehead (or other zone of interest) on the basis of the ambient temperature in the memory 209 or the temperature detected.

In a case where there is a temperature of above a certain threshold (for example a suspected fever) an alarm is emitted (for example a sound alarm, a visual alarm; means 204, 205).

Further, the temperature detected can be shown on a display 211.

It is worthy of note that in order to guarantee, in this case, correct pointing of the sensor 207, i.e. to identify and collect only the infrared radiation coming from the zone of interest (target area) of the patient, it will be for example possible to realise at least a portion of the front surface of the panel device 202 made of a reflective material (optionally mirrored) such that the user when nearing can see his or her own face, or at least the target area reflected.

There will be a reference sign 210 on the frontal surface, for example a light signal or possibly a mark or sign, drawn or reported, which will be visible to the user and this will be visible to the user, who will superpose it on the target area (i.e. by approaching the user will locate the reference sign on the target area).

In this case the subject must move such as to place the sign 210 at, for example, the centre of the forehead, or other significant target area, of his or her own reflected image.

Note that the apparatus 200 can be totally independent and different from the thermal imaging camera 1.

Alternatively the apparatus could be associated to other apparatus already in existence, such as for example metal detectors in airports, tribunals etc., and the measuring could be carried out even if the subject did not stop but simply slowed down and passed in a determined position and the system would be able to automatically calculate the temperature of the zone taken as a reference zone when the subject is at the right distance, or could automatically adjust it according to the distance (in a case in which the optimal distance cannot be reached, for example in the case of a child).

The possible use of a parabolic wave guide to carry the infrared radiation to the sensor might enable, if necessary, reduction of the field of view of the sensor and might make the measurement more accurate.

In a further aspect of the invention, a method is disclosed for measuring temperature where it is possible to use a thermometer realised as follows.

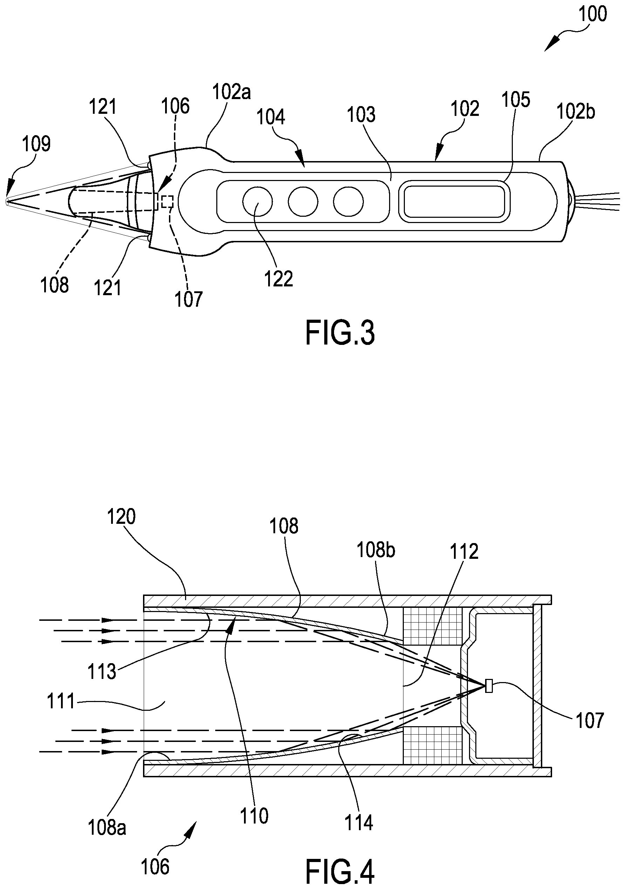

With reference to figure 3, an infrared thermometer 100 is illustrated in its entirety, according to a further aspect of the present invention. Conventionally the infrared thermometer comprises a main containing body 102 which defines a handling zone or grip 103 for a user.

The grip 103 can bear conventional command means 104, such as a keyboard or the like, as well as one or more displays 105 for a temperature reading and/or other data.

Sensor means 106 of infrared radiation are located at an end of the main body, which means 106 comprise a sensor organ of an infrared radiation intensity sensor 107 and at least a wave guide 108 operatively associated to the sensor organ such as to advantageously convey thereto the radiations emitted by the zone or target area 109 of the body the thermal level of which is to be measured.

In greater detail (figure 4) the wave guide 108 exhibits a first end 108a, facing towards the body the temperature of which is to be detected, and a second end 108b facing towards the sensor organ 107.

As can be noted from the accompanying figures, the wave guide is structured in the form of a tubular body exhibiting an internal surface 110, specular, which defines a passage able to set a first and a second opening 111 and 112, opposite one another, of the tubular body in optical communication.

The internal surface 110 of the wave guide has a convergent progression in a nearing direction to the second opening 112, i.e. it exhibits an internal diameter which progressively reduces as it proceeds from the first opening 11 of the wave guide 108 towards the second opening where the sensor organ 107 is substantially located.

More precisely, in accordance with the present invention, it is noteworthy that the convergence of the wave guide 108 is progressively more accentuated as it nears the second opening 112 of the tubular body.

In other words, the wave guide of the present invention can exhibit due or more tracts, axially consecutive to one another, exhibiting a respective convergence which is constant in each tract and progressively more accentuated in passing from one tract to a subsequent tract in a direction of and towards the second opening 112 of the tubular body defining the wave guide.

In practice, in the above-described case, at least the converging portion of the wave guide will appear as a succession of truncoconical surfaces with a conicity that is progressively more accentuated as it nears the second opening 112.

Alternatively, instead of two or preferably a plurality of progressively increasing consecutive converging tracts, a wave guide can be provided in which the internal surface is curved and progressively and continually converges, in an increasingly more marked way proceeding from the first opening towards the second opening.

In any case, the wave guide of the invention is realised in such a way that, given an equal axial nearing advancement to the second opening, there is a progressively greater diameter reduction proceeding from the first towards the second opening.

In the wave guide illustrated by way of example in the accompanying drawings, where longitudinal sections thereof are represented, the internal surface 110 of the wave guide is defined by arc lines 113, 114 and, preferably, by conical arcs having axes coinciding with the longitudinal axis of symmetry of the wave guide as well as having a concavity facing towards the first opening 11.

As can be seen, the convergence of the parabolic arcs is progressively greater proceeding towards the second opening 112.

The internal surface 110 has specular characteristics in order to be able to reflect the radiations incident there-upon.

In one of the possible embodiments the wave guide, at the first opening, can preferably and advantageously not exhibit any protection mask, like those typically provided on traditional wave guides for these uses, and must therefore be subjected to periodical cleaning by the users with the aim of guaranteeing the required performance.

Note that the possible absence of the protective mask is extremely advantageous as it prevents any useless loss of the radiation signal entering the wave guide.

In a case of a wave parabolic-shaped guide the sensor can be located substantially at the focus of the parabolic surface, with the aim of efficiently collecting in particular the radiations originating from parallel direction or slightly inclined with respect to the longitudinal axis of the wave guide.

Note that beyond the structure given to the wave guide, the wave guide as well as the sensor organ operatively associated thereto are typically housed internally of an auxiliary tubular body 120 made of a metal, preferably copper or zamak visible in particular in figure 4.

Also worthy of note is that the infrared thermometer of the invention, apart from having the above-described components, also comprises a processing unit (not illustrated) able to process the signal in output from the infrared radiation sensor organ and, by means of appropriate algorithms, to generate the thermal reading which is transferred to the display or shown to the user via other viewing systems, for example by projection, such as those described in application no. PCT/IT98/00379 in the name of the same Applicant.

The infrared thermometer of the present invention can further be provided with control means associated operatively to the containing body and cooperating with the processing unit; these control means are suitable for determining a correct positioning condition of the sensor organ 107 at a predetermined distance D from the detection area, which correct positioning distance is considered optimal for the performing of an accurate reading and for the delimitation of the reading area exclusively of the zone of interest.

It is clear that apart from the above-described special profiling described of the wave guide of the invention, a correct positioning at an adequate distance D between the sensor organ and the detection surface contributes to obtaining an extremely precise thermal reading.

At the level of realisation, the control means can be constituted by various technical solutions usable alone or in combination with one another.

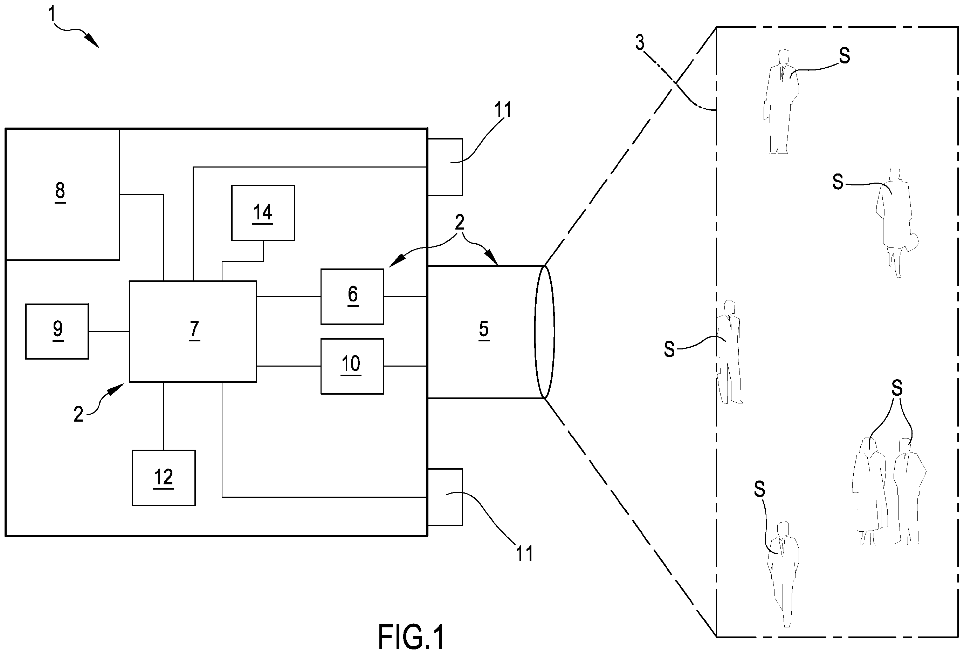

In particular, the use of luminous emitters or point lights 121 (see figure 1) can be comprised. In particular two or more visible luminous rays can be comprised, preferably not coplanar to one another and convergent. In greater detail, it is comprised that the luminous non coplanar rays converge towards a point on the target area such as to determine a figure (for example a spot or other) when the sensor organ is positioned at the correct distance D from the detecting area.

Alternatively, control means can be used constituted by an emitter of a luminous beam destined to be incident on the reading surface and to be reflected thereby in order to generate a return signal which is perceptible by a detecting organ which is able to calculate the inclination between the incident ray and the reflected ray. In particular one or more reading organs of the incident-reflected rays can be comprised, positioned in reciprocally symmetrically opposite positions and cooperating with the processing unit in order to calculate the inclination of the reflected ray and thus be able therefrom to derive the real distance of the sensor organ from the detection surface.

When the distance corresponds to the correct positioning distance D, optical/acoustic means can be comprised for signalling reaching of this condition to the user.

Once more as an alternative to the two above-described solutions, or contemporaneously there-with, the control means can comprise a generator of a light beam operating in such a way as to focalise a primary figure when the sensor organ 107 is at the correct positioning distance D.

Note that it is possible to comprise the use of a matrix optical selector, for example a liquid crystal type, able to vary the shape of the primary figure focalisable on the detecting surface. In greater detail, the matrix optical selector is managed by the central processing unit which is preferably able to vary the shape of the primary figure as a function of the temperature detected.

Note that a secondary light beam can be combined with the primary light beam, which secondary light beam is also variable with the use of a matrix optical selector, preferably of the liquid crystal type, manageable by CPU. The second secondary light beam can also be controlled such as to compose a secondary figure when the sensor organ is at the correct distance D from the detecting surface.

The secondary figure is also focalisable directly on the detecting surface and can be made up of the first figure and/or can delimit the reading area from which it is reasonably presumed that the sensor organ is reading the radiations and is therefore collecting the thermal information.

As a further variant, possibly also combinable with the three above-described solutions, the control means can lastly comprise an incident undulating signal emitter (acoustic and/or electromagnetic) which determines a return signal following reflection on the detecting surface. The central processing unit coordinates the emission and reception of the undulating signal by calculating a temporal difference between the two signals. It is clear that the temporal difference will be proportional to the distance of the thermometer and thus of the emitter from the surface of the body the thermal level of which is to be detected. In this way it is possible to know when the sensor organ is at the correct distance D from the detecting surface, which the CPU signals to the user via signalling means, for example optical or acoustic.

The infrared thermometer containing body preferably exhibits an elongate structure and comprises that the detecting portion and therefore the means for conveying the infrared radiation to the sensor organ are located at a first end 102a of the containing body.

After the above prevalently structural description, there now follows a description of the general functional operation of the wave guide and infrared thermometer according to the invention.

Thanks to the shape of the internal surface of the wave guide, which is progressively and ever more greatly convergent as it nears the sensor organ, substantially the following effects obtain: the radiations directed parallel to the longitudinal axis of the wave guide or slightly inclined with respect thereto are conveyed by the wave guide and substantially focalised on the sensor organ, independently of the zone in which they come into contact with the internal surface of the wave guide itself.

The rays coming from a zone of the patient which is not of interest and having an excessive inclination, which can falsify the thermal reading, can be sent back towards the inlet opening of the wave guide following multiple reflections (practically, for each reflection there is an increase in the inclination of the ray up to exceeding 90° with respect to the axis of the wave guide) and in this way the infrared radiation in inlet to the wave guide having an angle of greater than $\alpha_{max}$ is reflected externally of the device.

In general, thanks to the conformation of the internal surface of the wave guide, the rays having a greater inclination with respect to the longitudinal axis of the wave guide cannot reach the second opening of the wave guide; the only rays which can reach the second opening are small-inclination rays (which will strike the sensor organ) or rays which, according to the inclination, will break up on the internal walls or external walls of the containing body of the sensor organ.

It is however clear that thanks to the conformation given to the wave guide and the detecting portion, a considerable reduction and above all a precise definition of the effective target area for detecting the radiation on the surface of the body to be measured is obtained; in fact, the wave guide exerts a sort of optical filter of the radiations coming from excessively inclined directions with respect to the longitudinal axis of the guide.

Apart from performing a screening of the excessively-inclined rays, the wave guide also succeeds in better focalising substantially a majority of the rays coming from the reading area of interest, obtaining overall a signal which is sufficient to allow a thermal reading without radiations originating from zones which are not of interest and without an excessive reduction of the signal/noise ratio.

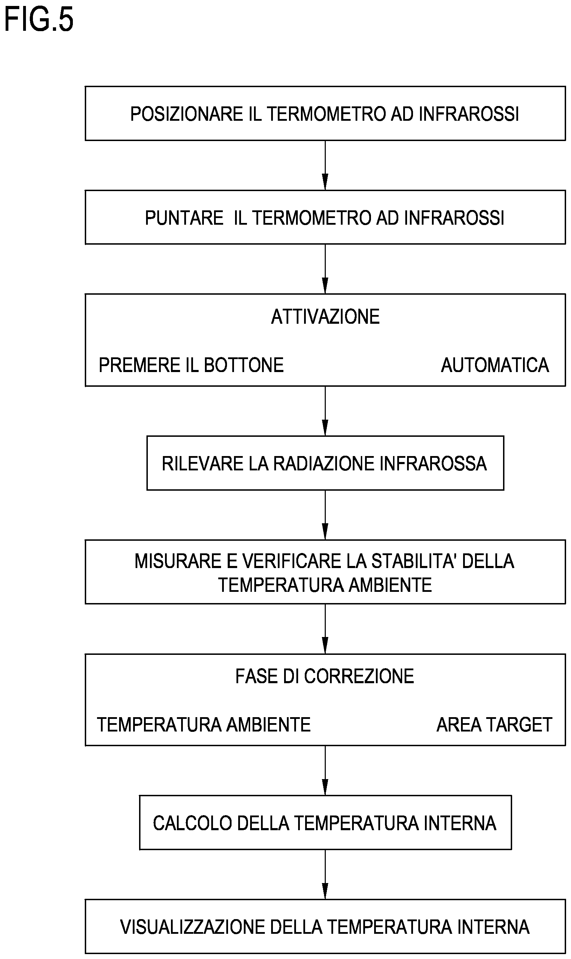

Given the above, the thermometer of figure 3 (but also the thermal imaging camera of figure 1) is particularly suited to measuring temperatures according to the method schematically illustrated in figure 5.

The method first comprises a step of manual positioning of the contactless infrared thermometer 10 at a predetermined distance D from a target area 109 of a patient/subject whose internal temperature is to be measured.

In particular, the thermometer will be hand-held at a gripping portion 103 thereof, for example directly by an operator.

Alternatively, and still using a contactless technology, the thermometer can be positioned fixed at a measuring zone and the patient/subject will be asked to near the zone such that, and still without contact, the thermometer can detect the temperature substantially in accordance with the diagram of figure 5.

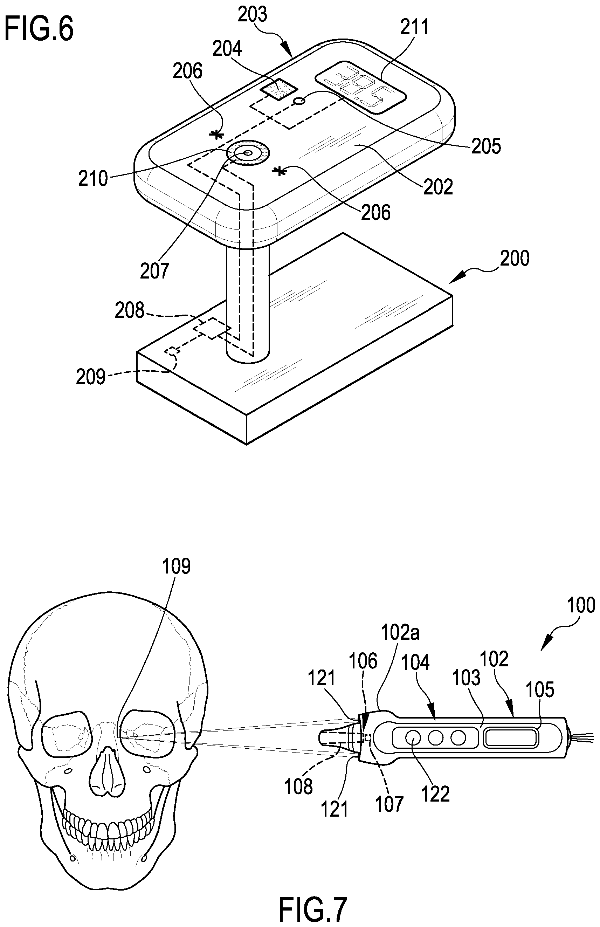

For example the thermal imaging camera of figure 1 can be fixed to a support and the subject S nears it (in all cases without contact with the thermometer). See for example figure 6.

As mentioned above, the thermometer is contactless with regard to the patient, i.e. not only is the infrared sensor 107 at a predetermined distance from the area in which the temperature is to be measured, but also no part of the instrument is in contact with the patient/subject during the step of measuring.

Reference will hereinafter be made to a specific embodiment, with a manually-utilised thermometer, without however intending to limit its use to only this situation.

The operator points the thermometer in the direction of the target area 109 of which the temperature is to be read, in particular in such a way that the infrared radiation coming from the target area can enter the wave guide 108 and be conveyed onto the infrared sensor 107 itself.

Once the correct distance D from the target area has been determined (note that this distance can be determined by way of example using optical methods such as non-planar converging rays, but also with a calculation of the sending and receiving time of a signal reflected by the target area such as ultrasound or other above-described methods), the infrared thermometer is activated to measure the internal temperature of the patient/subject.

In this case too the correct (relative) distance D can be achieved using a fixed thermometer and a subject who nears the sensor of the thermometer.

Alternatively, if the measuring system of the distance is able to determine the distance, the thermometer is able to determine the correct temperature, taking account of the distance at which the subject is located, whatever distance that may be (obviously within minimum and maximum limits of admissible distance).

The pointing of the target area can also be automatic rather than manual, i.e. the thermometer/thermal imaging camera will identify the specific target area by detecting (or considering) only the specified infrared radiation coming from that zone.

Note that the activation of the infrared thermometer will in general (even though not necessarily) be manual and will be triggered by pressing a button 122 present, for example, on a gripping portion 103 of the thermometer.

However, in particular situations, or with more evolved instruments, the activating of the temperature measurement can be done automatically and this activation can optionally be generated on reaching the correct distance.

In still further terms, once the correct positioning distance D of the thermometer has been determined, a control unit automatically initiates the reading of the infrared signal.

Once the thermostat has been activated, an infrared radiation coming from the target area 109 of the patient for a predetermined time interval is read; the intensity of infrared radiation detected makes it possible to calculate the temperature of the target area or equivalently the internal temperature of the patient.

The target area 109 advantageously used is the eyelid of a subject.

In particular, in a case in which the target area is the eyelid, the upper eyelid is selected and the method comprises in general that before the reading is done the patient is asked to close at least one eye such that the temperature of the closed upper eyelid can be read.

Obviously the upper eyelid is protected when the eyes are open and is also in contact with the eyeballs, and for this reason it is less subject to external temperature variations and is better stabilised with reference to the internal temperature of the patient.

Lastly, on activation of the thermometer, the reading of the infrared radiation for the mentioned predetermined time interval is determined by means of a countdown using a timer.

In other terms, once the thermometer has been activated the infrared sensor receiving radiations originating from the target area 109 transmits the intensity receive to the control unit, which initiates the calculation of the internal temperature of the patient.

The amount of infrared radiation is received and processed up to when the predetermined time interval has ended.

The further radiation received after the interval is not considered for the purposes of the calculation.

The invention provides important advantages.

The detected temperature is then shown on the display 105 which is in general back-lit.

The colour and/or intensity of the back-lighting can be a function of the type of measurement being made, for example it could have a first colour in a case of a temperature measurement of an object and a second colour, different from the first, in a case of measuring a temperature of a patient/subject.

Obviously the colours can also be more than two, associating the measurement to different situations: a colour for an object, a second colour for an animal (not a person), a third colour for a person, a further colour for evidencing an error situation or a need to repeat the reading, etc.

The different colour can be determined according to the button pressed for the measuring (in particular a first button for measuring inanimate objects, a second for human patients/subjects, a third for further uses, etc.), or according to the temperature level detected.

It is clear that the foregoing is equally applicable to the display 8 of the thermal imaging camera commanded by the processing unit 7, as well as for any eventual supplementary displays or other types of visualisation of the temperature detected such as for example projection on the forehead or other zones of the body or of objects.

The identification of specific target areas which enable a distance measuring of temperatures using contactless thermometers which however exhibit characteristics such as to maintain a more stabilised temperature even in poor ambient conditions improves the reading of the internal temperature and considerably increases the operating range of known thermometers.

**Claims**

1. A method for measuring a temperature, comprising following steps:

    positioning a contactless infrared thermometer at a predetermined distance from a target area of a patient, when it is desired to know the patient's internal body temperature;
    pointing at the target area such that an infrared radiation sensor of the thermometer can receive infrared radiations coming from the target area;
    activating the contactless infrared thermometer in order to measure the internal temperature of the patient;

    detecting, using the contactless infrared thermometer and for a determined time interval, an intensity of an infrared radiation coming at least from the target area of the subject;
    **characterised in that** the target area is a closed upper eyelid of the body of the human patient; the method including the step of closing at least an eye and detecting an intensity of an infrared radiation coming from the closed upper eyelid.

2. The method of claim 1, comprising a stage of correcting a temperature of the target area, which is a function of an intensity of infrared radiation detected, in order to determine the internal temperature.

3. The method of any one of the preceding claims, comprising a stage of supporting the thermometer manually during all the stages of positioning, pointing, activating and detecting.

4. The method of any one of claims 1 or 2, wherein the stage of positioning the thermometer at a predetermined distance is performed by nearing/distancing the subject from a thermometer in a fixed position and/or nearing/distancing the thermometer from the subject up to reaching the predetermined distance.

5. The method of any one of the preceding claims, comprising a step of measuring an ambient temperature and a step of correcting the temperature of the target area or the internal temperature as a function of the measured ambient temperature.

6. The method of claim 5, comprising a step of controlling a stability/correctness of the ambient temperature used for correcting the temperature of the target area or the internal temperature.

7. The method of any one of the preceding claims, wherein the step of activating the contactless infrared thermometer is a step of manually activating the thermometer by pressing at least a button located thereon.

8. The method of any one of the preceding claims, wherein after having activated the infrared thermometer, a timer begins a countdown, the detection of radiation being effected for a predetermined time during the count-down and up to the end of the count-down.

9. The method of any one of the preceding claims, wherein the contactless infrared thermometer is defined by a television camera, the step of pointing at the target area comprising sub-steps of acquiring an image coming from a predefined area, performing an identification of the target area, considering infrared radiation coming from only the target area for the purpose of determining the temperature.

**Patentansprüche**

1. Verfahren zum Messen einer Temperatur, umfassend die folgenden Schritte:

  Positionieren eines berührungsfreien Infrarotthermometers in einem vorbestimmten Abstand von einem Zielbereich eines Patienten, wenn es erwünscht ist, die innere Körpertemperatur des Patienten zu kennen;
  Zielen auf den Zielbereich, so dass ein Infrarot-Strahlungssensor des Thermometers Infrarotstrahlungen empfangen kann, welche von dem Zielbereich kommen;
  Aktivieren des berührungsfreien Infrarotthermometers, um die innere Temperatur des Patienten zu messen;
  Erfassen einer Intensität einer Infrarotstrahlung, welche wenigstens von dem Zielbereich der Testperson kommt, unter Verwendung des berührungsfreien Infrarotthermometers und für ein bestimmtes Zeitintervall;
  **dadurch gekennzeichnet, dass** der Zielbereich ein geschlossenes oberes Augenlid des Körpers des menschlichen Patienten ist; wobei das Verfahren den Schritt eines Schließens wenigstens eines Auges und eines Erfassens einer Intensität einer Infrarotstrahlung umfasst, welche von dem geschlossenen oberen Augenlid kommt.

2. Verfahren nach Anspruch 1, umfassend einen Schritt eines Korrigierens einer Temperatur des Zielbereichs, welche eine Funktion einer Intensität von erfasster Infrarotstrahlung ist, um die innere Temperatur zu bestimmen.

3. Verfahren nach einem der vorhergehenden Ansprüche, umfassend einen Schritt eines manuellen Unterstützens des Thermometers während all der Schritte des Positionierens, Zielens, Aktivierens und Erfassens.

4. Verfahren nach einem der Ansprüche 1 oder 2, wobei der Schritt des Positionierens des Thermometers in einem vorbestimmten Abstand durch ein Annähern/Entfernen der Testperson von einem Thermometer in einer festen Position und/oder ein Annähern/Entfernen des Thermometers von der Testperson bis zum Erreichen des vorbestimmten Abstands durchgeführt wird.

5. Verfahren nach einem der vorhergehenden Ansprüche, umfassend einen Schritt eines Messens einer Umgebungstemperatur und einen Schritt eines Korrigierens der Temperatur des Zielbereichs oder der inneren Temperatur als eine Funktion der gemessenen Umgebungstemperatur.

6. Verfahren nach Anspruch 5, umfassend einen Schritt eines Kontrollierens einer Stabilität/Korrektheit der Umgebungstemperatur, welche für das Korrigieren der Temperatur des Zielbereichs oder der inneren Temperatur verwendet wird.

7. Verfahren nach einem der vorhergehenden Ansprüche, wobei der Schritt des Aktivierens des berührungsfreien Infrarotthermometers ein Schritt eines manuellen Aktivierens des Thermometers durch Drücken von wenigstens einem Knopf ist, welcher daran angeordnet ist.

8. Verfahren nach einem der vorhergehenden Ansprüche, wobei, nachdem das Infrarotthermometer aktiviert worden ist, ein Zeitgeber einen Countdown beginnt, wobei die Erfassung der Strahlung für eine vorbestimmte Zeit während des Countdowns und bis zu dem Ende des Countdowns ausgeführt wird.

9. Verfahren nach einem der vorhergehenden Ansprüche, wobei das berührungsfreie Infrarotthermometer durch eine Fernsehkamera definiert ist, wobei der Schritt des Zielens auf den Zielbereich Unterschritte eines Aufnehmens eines Bildes, welches von einem vorbestimmten Bereich kommt, eines Durchführens einer Identifikation des Zielbereichs, eines Berücksichtigens von Infrarotstrahlung, welche von nur dem Zielbereich kommt, für den Zweck eines Bestimmens der Temperatur, umfasst.

**Revendications**

1. Procédé de mesure d'une température, comprenant les étapes suivantes :

  de positionnement d'un thermomètre infrarouge sans contact à une distance prédéterminée d'une zone cible d'un patient, lorsqu'il est souhaité connaître la température interne du corps du patient ;
  de pointage au niveau de la zone cible de sorte qu'un capteur de rayonnement infrarouge du thermomètre peut recevoir les rayonnements infrarouges provenant de la zone cible ;
  d'activation du thermomètre infrarouge sans contact afin de mesurer la température interne du patient ;
  de détection, en utilisant le thermomètre infrarouge sans contact et sur un intervalle de temps déterminé, d'une intensité d'un rayonnement infrarouge provenant d'au moins la zone cible du sujet ;

**caractérisé en ce que** la zone cible est une paupière supérieure fermée du corps du patient humain ; le procédé comprenant l'étape de fermeture d'au moins un oeil et la détection d'une intensité d'un rayonnement infrarouge provenant de la paupière supérieure fermée.

2. Procédé selon la revendication 1, comprenant un stade de correction d'une température de la zone cible, qui est une fonction d'une intensité du rayonnement infrarouge détecté, afin de déterminer la température interne.

3. Procédé selon l'une quelconque des revendications précédentes, comprenant un stade de soutien du thermomètre manuellement durant tous les stades de positionnement, de pointage, d'activation et de détection.

4. Procédé selon l'une quelconque des revendications 1 ou 2, le stade de positionnement du thermomètre à une distance prédéterminée étant exécuté en approchant/éloignant le sujet d'un thermomètre en une position fixe et/ou approchant/éloignant le thermomètre du sujet jusqu'à atteindre la distance prédéterminée.

5. Procédé selon l'une quelconque des revendications précédentes, comprenant une étape de mesure d'une température ambiante et une étape de correction de la température de la zone cible ou de la température interne comme fonction de la température ambiante mesurée.

6. Procédé selon la revendication 5, comprenant une étape de contrôle d'une stabilité/exactitude de la température ambiante utilisée pour corriger la température de la zone cible ou la température interne.

7. Procédé selon l'une quelconque des revendications précédentes, l'étape d'activation du thermomètre infrarouge sans contact étant une étape d'activation manuellement du thermomètre par pression d'au moins un bouton localisé dessus.

8. Procédé selon l'une quelconque des revendications précédentes, dans lequel après avoir activé le thermomètre infrarouge, un chronomètre commence un décompte, la détection du rayonnement étant effectué sur une durée prédéterminée durant le décompte et jusqu'à la fin du décompte.

9. Procédé selon l'une quelconque des revendications précédentes, le thermomètre infrarouge sans contact étant défini par une caméra de télévision, l'étape de pointage au niveau de la zone cible comprenant les sous-étapes d'acquisition d'une image provenant d'une zone prédéfinie, l'exécution d'une identification de la zone cible, la prise en considération du rayonnement infrarouge provenant uniquement de la zone cible aux fins de détermination de la température.

FIG.1

FIG.2

EP 2 577 242 B1

FIG.3

FIG.4

# FIG.5

```
┌─────────────────────────────────────────────────────┐
│        POSIZIONARE IL TERMOMETRO AD INFRAROSSI        │
└─────────────────────────────────────────────────────┘
                          │
                          ▼
┌─────────────────────────────────────────────────────┐
│         PUNTARE  IL TERMOMETRO AD INFRAROSSI          │
└─────────────────────────────────────────────────────┘
                          │
                          ▼
┌─────────────────────────────────────────────────────┐
│                     ATTIVAZIONE                       │
│                                                       │
│   PREMERE IL BOTTONE                    AUTOMATICA    │
└─────────────────────────────────────────────────────┘
                          │
                          ▼
        ┌───────────────────────────────────────┐
        │      RILEVARE LA RADIAZIONE INFRAROSSA │
        └───────────────────────────────────────┘
                          │
                          ▼
┌─────────────────────────────────────────────────────┐
│      MISURARE E VERIFICARE LA STABILITA' DELLA        │
│              TEMPERATURA AMBIENTE                      │
└─────────────────────────────────────────────────────┘
                          │
                          ▼
┌─────────────────────────────────────────────────────┐
│                 FASE DI CORREZIONE                    │
│                                                       │
│   TEMPERATURA AMBIENTE                  AREA TARGET   │
└─────────────────────────────────────────────────────┘
                          │
                          ▼
        ┌───────────────────────────────────────┐
        │   CALCOLO DELLA TEMPERATURA INTERNA    │
        └───────────────────────────────────────┘
                          │
                          ▼
┌─────────────────────────────────────────────────────┐
│     VISUALIZZAZIONE DELLA TEMPERATURA INTERNA         │
└─────────────────────────────────────────────────────┘
```

FIG.6

FIG.7

FIG.8

FIG.9

**EP 2 577 242 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102004027443 B3
- US 2004254472 A
- US 6940545 B
- US 7218759 B
- US 20090190803 A
- IT 9800379 W